# EUROPÄISCHE PATENTANMELDUNG

(11) **EP 2 255 821 A1**
(43) Veröffentlichungstag der Anmeldung: **01.12.2010**
(21) Anmeldenummer: 10158892.9
(22) Anmeldetag: 28.02.2003
(51) Int. Cl.: A61K 35/74, A61K 9/16, A61K 9/51

(54) **Mikro-/Nanopartikel aus lipidhaltigen marinen Organismen für Pharmazie und Kosmetik**

(30) Priorität: 28.02.2002 DE 10208949; 24.08.2002 DE 10208364
(62) Teilanmeldung aus: 03720153.0
(71) Anmelder: Heitland & Petre International GmbH, 29229 Celle (DE); Elastèn Pharmavertriebs GmbH, 21465 Reinbek (DE)
(72) Erfinder: Lukowski, Gerold, D-17489, Greifswald (DE); Jülich, Wolf-Dieter, D-17489, Greifswald (DE); Lindequist, Ulrike, D-17491, Greifswald (DE); Mundt, Sabine, D-18439, Stralsund (DE)
(74) Vertreter: Wehlan, Helmut

(57) **Zusammenfassung**

Die Erfindung betrifft pharmazeutisch wirksame Mittel, die durch Umwandlung von Biomassen lipidhaltiger mariner Organismen in Mikro- und Nanopartikel gewonnen wurden und vorzugsweise einen mittleren Durchmesser von 10 nm -10 µm besitzen. Mögliche Anwendungsgebiete sind die Medizin oder die Nahrungsmittelherstellung, insbesondere Prophylaxe nosokomialer Infektionen, Beschleunigung des Zellwachstums und Hemmung von Staphylokokken.

## Beschreibung

Die Erfindung betrifft pharmazeutisch wirksame Mittel, die durch Umwandlung von Biomassen lipidhaltiger mariner Organismen in Mikro- und Nanopartikel gewonnen wurden und vorzugsweise einen mittleren Durchmesser von 10 nm bis 10 µm besitzen. Mögliche Anwendungsgebiete sind die Medizin oder die Nahrungsmittelherstellung.

Marine Lebewesen, insbesondere Mikro- und Makroalgen, marine Pilze (Thraustochytriden) und marine Bakterien, zeichnen sich durch einen hohen Gehalt an Lipiden und eine spezifische Lipidzusammensetzung aus (Lindequist,U. and Th. Schweder: Marine Biotechnology, in: Biotechnology. 2. ed., Ed. H.-J.Rehm Wiley-VCH Weinheim 2001, pp. 442-473).

So können z.B. Mikroalgen unter wachstumslimitierten Bedingungen, besonders unter N-Limitierung, bis zu 70 % des assimilierten Kohlenstoffs als energetisch effiziente Lipide speichern. Gehalt und Zusammensetzung der Lipide können durch die Wachstumsbedingungen beeinflusst werden. Während höhere Pflanzen vorwiegend Lipide mit relativ kurzkettigen Fettsäuren (C12-C18) und einer geringen Anzahl von Doppelbindungen (bis C18:3) produzieren, ist die Variabilität der von marinen Organismen gebildeten Fettsäuren ungleich höher. Algen synthetisieren zahlreiche mehrfach ungesättigte C16-C22-Fettsäuren, z.B. Linolsäure (C18:2), Linolensäure (C18:3), Arachidonsäure (C20:4), Eicosapentaensäure (EPA, C20:5) und Docosahexaensäure (DHA, C22:6) (Pohl,P. und F.Zurheide: Fatty acids and lipids of marine algae and the control of their biosynthesis by environmental factors. In: Marine Algae in Pharmaceutical Science. Ed. H.A.Hoppe, T.Levring, Y.Tanaka, Walter de Gruyter New York, 1979, pp. 473-523; Roessler,P.G.: Environmental control of glycerolipid metabolism in microalgae: commercial implications and future research directions. J. Phycology 26, 393-399, 1990; Puls, O.: Biotechnology with cyanobacteria and microalgae, in: Biotechnology, 2. ed., Ed. H.-J.Rehm Wiley-VCH Weinheim 2001, pp. 105-136, Servel,M.O., C.Claire, A.Derrien, L.Ciffard, Y.DeRoeck-Holtzhauer: Fatty acid composition of some marine microalgae. Phytochemistry 36, 691-693, 1994). Die heterotrophe Mikroalge Cryptecodinium cohnii kann bis zu 13 % ihrer Zellmasse als Lipide mit 36-43% DHA bilden (DeSwaaf, M.E., DeRijk, T.C., Eggink, G., Sijtsma, L.: Optimisation of docosahexaenoic acid production in batch cultivations by Crypthecodinium cohnii. J. Biotech. 70, 185 - 192, 1999).

Thraustochytriden sind pilzliche Protisten, die bis zu 50 % ihrer Lipide als DHA produzieren und im Cytoplasma in Form von Öltropfen ablagern (Lewis, T.E., Nichols, P.D., McMeekin, T.A.: The biotechnological potential of thraustochytrids, Mar. Biotechnol. 1, 580 - 587 1999).

Marine Bakterien inkorporieren im Gegensatz dazu die polyungesättigten Fettsäuren in Membranphospholipide. Der Gehalt an EPA in den Gesamtlipiden von Colwellia psychrerythrea liegt bei 6-7 % (Bowman, J.P., Gosink, J.J., McCammon,S.A., Lewis,T.T., Nichols,D.S., Skerratt, J.H., Rea, S.M., McMeekin, T.A.: Colwellia demingae sp. nov., Colwellia hornerae sp. nov., Colwellia rossensis sp. nov. and Colwellia psychrotropica sp. nov.: psychrophilic Antarctic species with the ability to synthesise docosahexaenoic acid (22:6 n-3), Int. J. Syst. Bacteriol. 48, 1171 - 1180, 1997).

Linol- (Z,Z-9,12-Octadecadiensäure) und Linolensäure (Z,Z,Z-9,12,15-Octadecatriensäure) sind als essentielle Fettsäuren für die menschliche Ernährung unersetzlich. Arachidonsäure (5,8,11,14-Eicosatetraensäure) ist Vorstufe der physiologisch wichtigen Eicosanoide. EPA und DHA (Omega-3-Fettsäuren) sind nicht nur für die frühkindliche Gehirnentwicklung notwendig, sondern besitzen auch prophylaktischen/therapeutischen Nutzen bei Herz-Kreislauf-Krankheiten, rheumatischen Erkrankungen, chronisch-entzündlichen Darmerkrankungen, Neurodermitis, Psoriasis und Allergien. Es wurde z.B. nachgewiesen, dass das Sterberisiko von Patienten nach einem bereits erlittenen Herzinfarkt durch tägliche Einnahme von Omega-3-Fettsäuren signifikant erniedrigt wird (GISSI-Studie). Von besonderem Interesse sind strukturell ungewöhnliche Fettsäuren, die über weitere biologische Eigenschaften verfügen. Die Hydroxyfettsäuren Coriolsäure (13-HODE *Z,E*) und Dimorphecolsäure (9-HODE *E,Z*), die aus einer Oscillatoria-Art isoliert wurden, zeichnen sich ebenso wie Linolensäure durch antibakterielle Aktivität aus (Kreitlow,S.: Dissertation Greifswald 2000).

Zusätzlich zu den Fettsäuren sind die marinen Organismen auch durch ihren Reichtum an Proteinen, Vitaminen und Mineralstoffen für Ernährung, Kosmetik und Medizin von Interesse [Lindequist,U. and Th. Schweder: Marine Biotechnology, in: Biotechnology. 2. ed., Ed. H.-J.Rehm Wiley-VCH Weinheim 2001, pp. 442-473; Puls, O.: Biotechnology with cyanobacteria and microalgae, in: Biotechnology, 2. ed., Ed. H.-J.Rehm Wiley-VCH Weinheim 2001, pp. 105-136, Becker, Microalgae:Biotechnology and Microbiology, Cambridge, 1994; Köhler, Kurth, Pulz, Spirulina platensis-an microalgal additiv for cosmetics, Biotechnology for Microalgae, 1997).

Die Herstellung und Anwendung von Extrakten aus Algen und die Anwendung von Algenbiomasse in den Bereichen Ernährung, Kosmetik und Medizin ist aus der Patentliteratur bekannt (z. B. US 4,320,050 A, IL 59766, DE 100 59 107 A1). Auch wurden spezielle Kombinationen von Fibrillin mit Blaualgenextrakten in der Kosmetik und Medizin beschrieben (WO 01/07006 A1). Weitere Patente beziehen sich auf die Produktion von polyungesättigten Fettsäuren durch marine Organismen (z.B. Barclay, W.R. (1992) Process for the heterotrophic production of microbial oils with high concentrations of omega-3 highly unsaturated fatty acids, U.S. Patent 5,130,242 A; Barclay, W.R. (1994) Process for growing Thraustochytrium and Schizochyrium using non-chloride salts to produce a micro-floral biomass having omega-3 highly unsaturated fatty acids, U.S. Patent 5,340,742 A).

Die Verwendung der lipidhaltigen Biomasse von marinen Organismen als Wirkstoffträger wurde bisher nicht beschrieben.

Bei der Herstellung von Nano- und Mikropartikeln sind verschiedene Herstellungsverfahren auf Basis von Lipiden oder Polymeren bereits bekannt. So wurden bereits Verfahren zur Herstellung von Lipid-Mikropartikeln auf Basis von Phospholipiden beschrieben, die antimykotische Eigenschaften haben und im pharmazeutischen oder kosmetischen Bereich eingesetzt werden können (DE 69 00 2905 T2). Andere Verfahren beschreiben Lipidnanopartikel auf Basis von extrahierten Mono-, Di und Triglyceriden, Ölen oder Wachsen. Mit Hilfe dieser gewonnenen Lipide werden ebenfalls pharmazeutische Wirkstoffe verkapselt (WO 94/20072 A1). Weitere Lipidnanopartikel beschreiben Substanzen ebenfalls auf Basis von Lipiden zur parenteralen Anwendung (WO 98/56362 A1). Auch werden spezielle Techniken zur Herstellung von Lipidnanopartikeln vorgestellt (z. B. EP 0 526 666 A). Bisher sind in der Literatur jedoch keine lipidhaltigen Mikro- und Nanopartikel auf Basis mariner Organismen (Mikro- und Makroalgen, Thraustochytriden, marine Bakterien) und deren Herstellung beschrieben worden.

Staphylococcus aureus ist ein häufiger Kommensale. Etwa 30% der Bevölkerung sind dauerhaft symptomfrei mit S. aureus besiedelt Die häufige symptomfreie Besiedlung führt zu einer oft unbemerkten Weiterverbreitung.

Andererseits ist S. aureus auch ein häufiger Erreger schwerer Infektionen und von Sepsis, vor allem bei abwehrgeschwächten Patienten.

Besorgniserregend ist die rasche Entwicklung multiresistenter S. aureus Stämme (MRSA). Auf Grund der Multiresistenz sind Infektionen mit MRSA therapeutisch schwer beherrschbar.

Die Verbreitung der MRSA-Stämme ist bisher jedoch weitgehend auf die Krankenhäuser beschränkt ist. Deshalb besteht unabhängig von der Ersterkrankung bei Aufnahme in ein Krankenhaus eine große Gefahr. Etwa 10 % der aufgenommenen Patienten erkranken an nosokomialen Infektionen. Es wird mit 600 000 bis 800 000 Infektionen mit multiresistenten Erregern gerechnet, die im Krankenhaus erworben werden (Hyg. Med. 26 (2001)183). Staphylokokken sind die wichtigsten Erreger, bei denen mit einem völligen Versagen der antibiotischen Therapie gerechnet wird. Eine Hautpflege, die der Besiedlung mit multiresistenten Keinem entgegenwirkt, kann bei der Aufnahme in ein Krankenhaus die Gefahr einer nosokomialen Infektion vermindern. Wenn nur ein Teil dieser Infektionen verhindern werden kann, wäre dies ein großer Erfolg.

Zur Dekolonisation beim Auftreten von MRSA werden bisher bakterizid wirkende Substanzen eingesetzt. Dadurch wird auch die Standortflora abgetötet. Damit wird bei einer Neubesiedlung der Haut die Ansiedlung von MRSA sogar erleichtert.

Kürzlich wurde bekannt, dass antimikrobielle Peptide die Haut vor einer invasiven bakteriellen Infektion schützen (V.Nizet, T. Ohtake, X. Lauth, J. Trowbridge, J. Rusdill, R.A. Dorschner, V. Pestonjamasp, J. Piraino, K. Huttner, R.L. Gallo: Innate antimicrobial peptide protects the skin from invasive bacterial infection. Nature 414, 454-457 (2001). Diese Peptide haben werden auf der Oberfläche von Epithelzellen und in Neutrophilen gebildet und bewirken eine sehr frühe Abwehr eingedrungener Erreger. Sie bewirken zelluläre Effekte, die sich deutlich von der antibakteriellen Aktivität in vitro unterscheiden. Der kritische erste Schritt einer Kausalkette, die letztendlich zu einer Infektion führt, ist die Bindung von Adhäsionen des Erregers an geeignete Rezeptoren auf der Hautoberfläche. Die Adhäsion wird in vielen Fällen eingeleitet durch Bindung von auf der Oberfläche des Erregers ausgebildeten Proteinen an Kohlenhydrat-Rezeptoren des Wirts, daneben spielen auch Protein-Protein-Wechselwirkungen und Bindung von Polysachariden des Erregers an Rezeptoren auf der Haut eine Rolle (Relman D, Tuomanen E, Falkow S et al., Cell 1990; 61: 1375-1382; Kanbe T., Cutler JE. Infect Immun 1994; 62: 1662-1668).

Aufgabe der vorliegenden Erfindung ist es daher, neue Wirkstoffe und Wirkstoffträger mit gegenüber dem Stand der Technik verbesserten Eigenschaften für verschiedene Zwecke bereitzustellen.

Die Aufgabe wurde durch pharmazeutisch wirksame Mittel aus marinen Organismen (Mikro- und Makroalgen, Thraustochytriden, marine Bakterien), die durch Unwandlung der Biomassen dieser Organismen in Mikro- und Nanopartikel erhalten wurden, gelöst. Erfindungsgemäß wurden Biomassen aus marinen Organismen (Mikro- und Makroalgen, Thraustochytriden, marine Bakterien) auf kostengünstigem direktem Weg zu den neuartigen Substanzen mit speziellen Wirkungen umgesetzt. Überraschenderweise hat sich herausgestellt, dass es gelungen ist, die gesundheitsfördernden Inhaltsstoffe der lipidhaltigen marinen Organismen besonders effizient zu nutzen und verschiedene Anwendungen zu ermöglichen, die mit den nativen Biomassen nicht erreicht werden können.

Das erfindungsgemäße Verfahren führt zu neuen, wertvollen Produkten, die auf bekannten Wegen nicht erreicht werden können und enthält drei Alternativen:

### 1. Homogenisationsverfahren

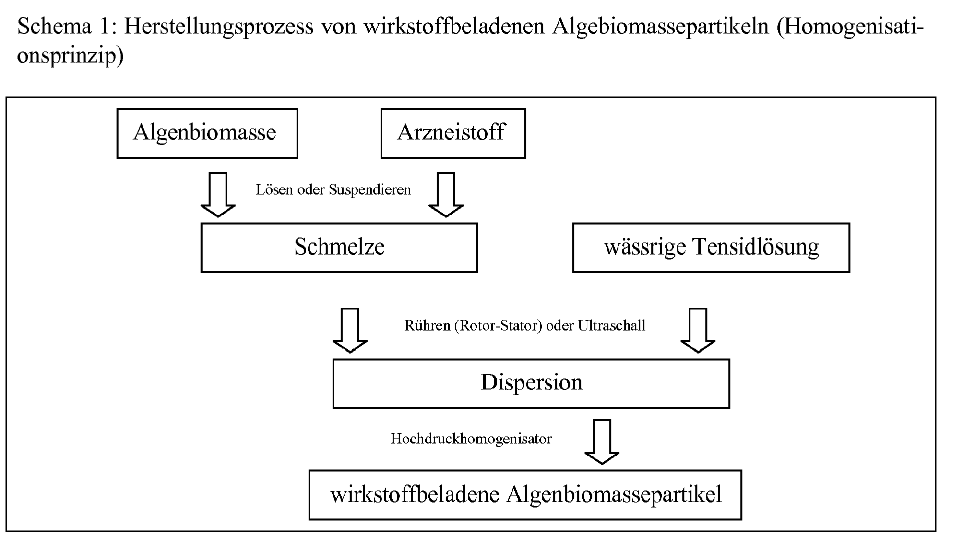

Die lipidhaltigen marinen Mikroorganismen werden zunächst erwärmt, so dass eine Verflüssigung der darin enthaltenen Fettsäuren erreicht wird. In diese Biomasse werden ein oder mehrere Wirkstoffe (fest oder flüssig) hinzugegeben (Schema 1). Der Wirkstoff wird in den Fettsäuren der Cyanobakterien bzw. der gesamten lipidhaltigen marinen Mikroorganismen suspendiert, dispergiert bzw. adsorbiert. Parallel dazu wird ein Tensid-Wasser-Gemisch hergestellt. Dieses Tensid-Wasser-Gemisch wird auf eine Temperatur oberhalb der Schmelztemperatur der Fettsäuren erwärmt. Die beiden Phasen werden bei der gewählten Temperatur vereint. Anschließend wird mit Hilfe eines Rührers (Rotor-Stator-Prinzip) oder mit Hilfe von Ultraschall eine Vorsuspension hergestellt. Die Vorsuspension wird danach mit Hilfe eines Hochdruckhomogenisators homogenisiert, wobei die Zahl der Homogenisationszyklen und der Arbeitsdruck nach der erwünschten Partikelgröße und Stabilität der Zubereitung gewählt werden. Zwischen den einzelnen Zyklen ist darauf zu achten, dass die Herstellungstemperatur immer wieder eingestellt wird. Das Tensid dient zur Stabilisierung der Suspension.

Sollte es bei der Herstellung Probleme mit der Höhe der Temperatur (z. B. empfindliche Wirkstoffe) geben, so besteht die Möglichkeit, das gesamte Verfahren auch bei Raumtemperatur durchzuführen. In diesem Falle wird das Verfahren in gleicher Weise, wie zuvor beschrieben, durchgeführt, wobei der Wirkstoff an den lipidhaltigen marinen Mikroorganismen adsorbiert oder bei Zusatz einer geringen Wassermenge dispergiert wird.

### 2. Lösungsmittel-Homogenisations-Verfahren

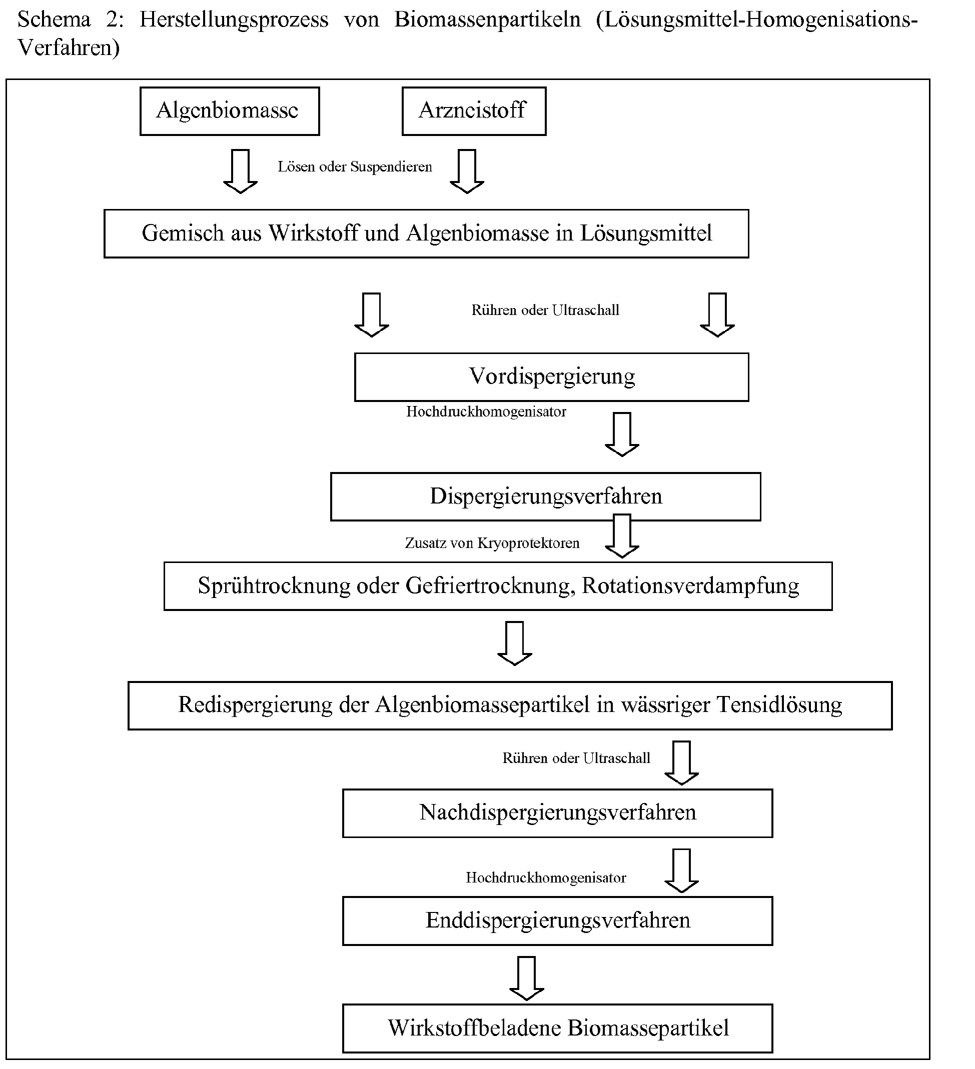

Die lipidhaltigen marinen Mikroorganismen und der Wirkstoff werden in einem verdampfbaren organischen Lösungsmittel suspendiert. Danach wird dieses Gemisch vordispergiert (Stator-Rotor-Prinzip oder Ultraschall), homogenisiert (Hochdruckhomogenisator) und anschließend sprühgetrocknet oder gefriergetrocknet (Schema 2). Beim Gefriertrocknen ist zu beachten, dass geeignete Kryoprotektoren eingesetzt werden. Es besteht darüber hinaus auch die Möglichkeit, das organische Lösungsmittel durch geeignete Verdampfer (z. B. Rotationsverdampfer) zu entfernen. Anschließend können die Partikel aus lipidhaltigen marinen Mikroorganismen in geeigneten wässrigen Tensid-Lösungen redispergiert werden. Danach ist eine erneute Dispergierung (Stator-Rotor-Prinzip oder Ultraschall) und Homogenisierung (Hochdruckhomogenisator) notwendig.

### 3. Lösungsmittel-Emulsions-Verfahren

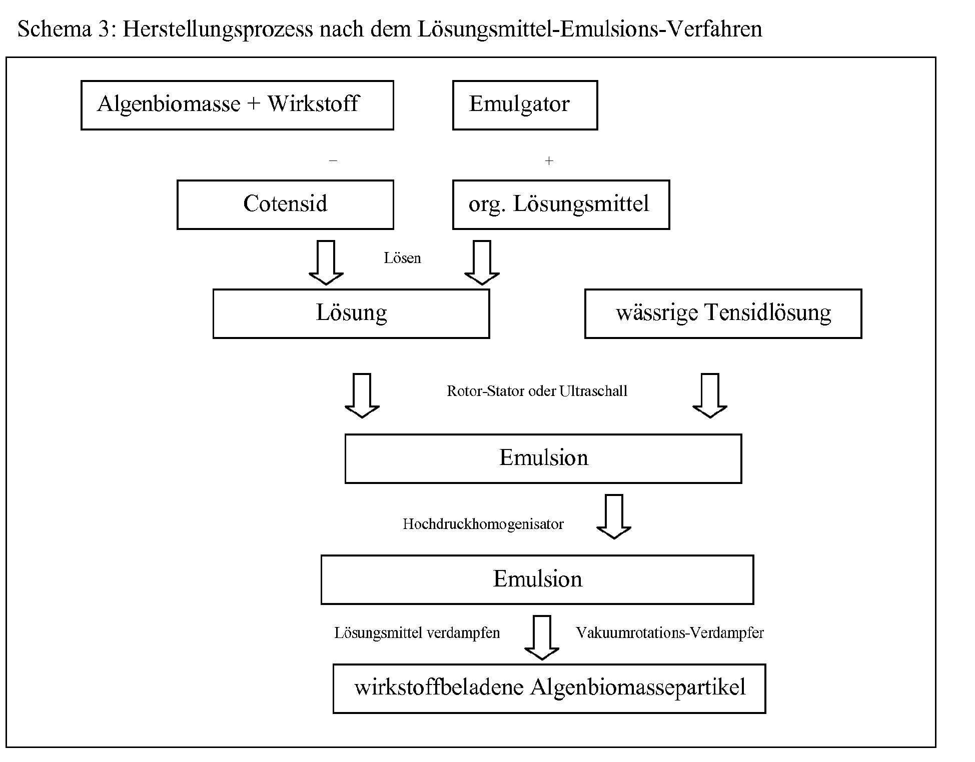

Dieses Verfahren basiert auf der Bereitung einer Emulsion aus Wasser und einer Lösung der Algenbiomasse-Wirkstoff in einem geeigneten organischen Lösungsmittel (Schema 3). Dazu wird ein Emulgator zur Dispergierung des Algenbiomasse-Wirkstoffes eingesetzt. Emulgator und Algenbiomasse werden in einem geeigneten organischen Lösungsmittel gelöst. Zu dieser Lösung wird eine wässrige Phase, die ein wasserlösliches Cotensid enthält, hinzugefügt. Danach wird dieses Gemisch vordispergiert (Stator-Rotor-Prinzip oder Ultraschall). Nach einem Homogenisationsschritt mit Hilfe eines Hochdruckhomogenisators wird das organische Lösungsmittel durch Verdampfen entfernt, wobei die Wirkstoff enthaltende Biomasse in Form von festen Partikeln ausfällt.

Die Anwendung dieser Verfahren führt zu neuartigen marinen Biomasse- Wirkstoff - Partikeln mit einem mittleren Durchmesser, der je nach Herstellungsart zwischen 10 nm und 10 µm liegt.

Die erfindungsgemäßen Mittel sind auch ohne die Zugabe von einem zusätzlichen Wirkstoff wirksam, weil durch das erfindungsgemäße Verfahren die Bestandteile der lipidhaltigen marinen Organismen besser verfügbar gemacht werden.

So war bereits überraschend, dass durch das erfindungsgemäße Verfahren nicht bakterizid wirkende natürliche Substanzen, die Inhaltsstoffe von marinen Organismen sind, z.B. Norlichhexanthon, nun in vitro das Wachstum von MRSA hemmen.

Außerdem war überraschend, dass nicht bakterizid wirkende natürliche Wirkstoffe wie die in der Natur weit verbreiteten Ubichinonderivate oder hydroxylierte Aromaten, wie sie in verschiedenen marinen Pilzarten gefunden werden, durch das erfindungsgemäße Verfahren ebenfalls in vitro das Wachstum von MRSA hemmen. Ein Einsatz dieser an sich bekannten Stoffe zur Bekämpfung von MRSA wurde bisher nicht beschrieben. Außerdem ist bekannt, dass die genannten Naturstoffe auf der Haut Staphylokokken nicht in ausreichendem Maße abtöten. Durch das erfindungsgemäße Verfahren wurde somit ein synergistischer Effekt erreicht.

Bei Übertragungsversuchen von mit MRSA kontaminierter Haut (Donator) auf keimarme Haut (Akzeptor) hat sich überraschenderweise gezeigt, dass eine Übertragung von MRSA weitgehend vermieden werden kann, wenn die Haut vor der Übertragung mit einem synergistisch wirkenden Kombination von Lipiden (als Bestandteile von marinen Organismen), Immunstimulantien, Radikalfängern und Xanthonen der allgemeinen Formel gepflegt wurde. Offensichtlich werden durch diese synergistische Kombination bereits sehr frühe Stufen der Kausalkette von der Übertragung der pathogenen Mikroorganismen bis zur Infektion gestört, so dass bereits die Ausbildung einer Kolonisation verhindert werden kann.

Spezielle Eigenschaften werden erreicht, wenn in den nach Anspruch 1 hergestellten Mikro- und Nanopartikel Aggregate aus den lipidhaltigen marinen Organismen und Tonmineralien (Phytosilikate) enthalten sind. Spezielle Charakteristika dieser Mikro- und Nanopartikel sind ihre große Oberfläche, ihr hohes Ionenaustauschvermögen, ihre große Quellfähigkeit und ihre Fähigkeit, in die Schichten der Silikatstruktur die unterschiedlichsten Wirkstoffe einzulagern. Überraschenderweise wurde gefunden, dass die auf diese Weise hergestellten Aggregate das Zellwachstum deutlich begünstigen.

Vorteilhaft ist insbesondere die Verwendung von Tonen, die in ihrer Kristallstruktur dem Bentonit entsprechen. Bentonit weist nicht nur einen sehr hohen Anteil von Partikeln mit einer Größe < 2 µm auf, sondern besitzt auch eine hohe Ionenaustausch Kapazität von 0,76 meq/g, eine extrem große spezifische Oberfläche von 562 m²/g und den stärksten Einfluss auf das Wachstum von Amnion-Epithelzellen.

Eine besonders vorteilhafte Ausführungsform der Erfindung ist die Verwendung von lipidhaltigen marinen Organismen, die z.B. in Gegenwart von Tonmineralien kultiviert wurden. Aufgrund der großen spezifischen Oberfläche sind auf dieser Basis hergestellte Mikro- und Nanopartikel als Wirkstoffträger hervorragend geeignet, zumal die Wirkstoffabgabe durch den Anteil und die Zusammensetzung der mineralischen Komponente sehr gut gesteuert werden kann.

Die vorgestellten Prinzipien sind geeignet, verschiedene pflegende Komponenten und/oder Mineralstoffe und/oder Radikalfänger und/oder Vitamine und/oder Nahrungsergänzungsstoffe in Biomassen zu einzulagern. Auf diese Weise ist es möglich, in der Kosmetik die wertvollen Inhaltsstoffe der Algen wie Proteine, Mineralien und Vitaminen, mehrfach ungesättigte Fettsäuren wie y-Linolensäure, Arachidonsäure, Eicosapentaensäure und Docosahexaensäure in besonders günstiger Form zu nutzen.

Gegenstand der Erfindung ist somit auch eine Zusammensetzung aus Biomassen mariner Organismen und Mineralstoffen und/oder Radikalfängern und/oder Nahrungsergänzungsstoffen und/oder Vitaminen, insbesondere Vitamin C.

Zusätzlich können erfindungsgemäß ein oder mehrere pharmazeutische Wirkstoffe in die Mikro- und Nanopartikel inkorporiert werden.

Als vorteilhaft hat sich der Zusatz von Xanthon (z.B. Norlichhexanthon) oder deren Derivaten und/oder Ubichinonen der Kettenlänge n= 1 bis n = 15 und/oder anorganischen Thiocyanaten und/oder Hydrothiocyanaten organischer Basen und/oder Trihydroxybenzaldehyd oder dessen Derivaten erwiesen.

Erfindungsgemäß können Biomassen mariner Organismen in Kombination mit Thiocyanaten und/oder Hydrothiocyanaten organischer Basen und/oder Trihydroxybenzaldehyd oder dessen Derivaten auch ohne die Umwandlung in Mikro- und Nanopartikel eingesetzt und als pharmazeutische Mittel verwendet werden oder aber zur Herstellung pharmazeutischer Mittel dienen.

Die erfindungsgemäße Herstellung hat den Vorteil, dass die Freisetzung der inkorporierten Substanzen durch Wahl der Temperatur, der Wirkstoffe, des Tonmineralanteils und der Tenside gesteuert werden kann. Erfindungsgemäß ist es vorteilhaft, die Teilchen in destilliertem Wasser oder in einem wässrigen Medium mit Zusätzen wie Elektrolyten, Polyonen, Mono-, Di- und Polysacchariden, Isotonisierungsmitteln, Puffersubstanzen, Gefrierschutzmitteln und Konservierungsmittel zu dispergieren. Um den erfindungsgemäßen Zweck zu erreichen, kann ein Zusatz von einem oder mehreren dispersionsstabilisierenden Substanzen notwendig sein. Eine vorteilhafte Ausführungsform ist **dadurch gekennzeichnet, dass** der Biomasse ein oder mehre Wirkstoffe, Vitamine oder Nahrungsergänzungsstoffe in fester und/ oder flüssiger Form zugesetzt werden. Zweckmäßig ist es, die zugesetzten Wirkstoffe in den Fettsäuren der Biomasse zu suspendieren, zu dispergieren oder zu adsorbieren. Erfindungsgemäß wird die Biomasse in einem weiteren Herstellungsschritt mit einem Tensid- Wasser-Gemisch vereinigt. Zweckmäßig ist es, zunächst mit Hilfe eines Rührers (Rotor-Stator-Prinzip) oder mit Hilfe von Ultraschall eine Vorsuspension herzustellen. Erfindungsgemäß wird diese Vorsuspension danach mit Hilfe eines Hochdruckhomogenisators homogenisiert, wobei die Zahl der Homogenisationszyklen und der Arbeitsdruck nach der dem erfinderischen Zweck entsprechenden Partikelgröße und Stabilität der Zubereitung gewählt werden.

Bei einem anderen, ebenfalls erfindungsgemäßen Herstellungsverfahren werden Biomasse und Wirkstoffe in einem verdampfbaren organischen Lösungsmittel suspendiert. Danach wird dieses Gemisch vordispergiert (Stator-Rotor-Prinzip oder Ultraschall) und homogenisiert (Hochdruckhomogenisator).Anschließend wird das Lösungsmittel durch Sprühtrocknung oder Gefriertrocknung oder mittels Rotationsverdampfer entfernt. Falls erforderlich, kann die Biomasse in geeigneten wässrigen Tensid-Lösungen redispergiert und danach nachdispergiert (Stator-Rotor-Prinzip oder Ultraschall) und anschließend homogenisiert (Hochdruckhomogenisator) werden. Falls erforderlich, kann ein Coemulgator bzw. ein Emulgator zur Dispergierung des Biomasse-Wirkstoffes-Mischung eingesetzt werden.

Die nach den beschriebenen Verfahren hergestellten Mikro und Nanopartikel ermöglichen eine Verwendung auf den verschiedensten Gebieten. Überraschenderweise zeigte sich eine deutlich bessere Bioverfügbarkeit der neuartigen Substanzen gegenüber den reinen Stoffen. Das ermöglicht die Verwendung der Partikel als pflegende Komponenten in kosmetischen Produkten allein oder in Kombination mit anderen Pflegeprodukten. Die neuen Substanzen können problemlos in andere Pflegekomplexgrundlagen eingearbeitet werden Die erfindungsgemäßen Formulierungen machen die Haut besonders geschmeidig und pflegen sie. Wie Fettemulsionen zeigen die Partikeln nur eine geringe systemische Toxizität und kaum Zytotoxizität.

Völlig überraschend wurde gefunden, dass Extrakte der Biomasse antibakterielle Effekte zeigen und in vitro sogar das Wachstum der multiresistenten Staphylokokken deutlich reduzierten. Nach Umwandlung in Mikro- und Nanopartikel unterstützen die Inhaltstoffe der marinen Organismen die natürliche Abwehrbarriere der Haut. Die Bindung einer pathogenen Mikrobe an einen Rezeptor des Wirtes ist der kritische frühe Schritt in einer Entwicklung zu einer Kolonisation bzw. Infektion. Bereits dieser frühe Schritt kann überraschenderweise durch die erfindungsgemäßen Formulierungen beeinflusst werden.

Die vorliegende Erfindung ermöglicht erstmals die Verwendung lipidhaltiger mariner Organismen als Wirkstoffträger, z.B. für Antibiotika.

Die Erfindung erlaubt die Verwendung von Biomassen lipidhaltiger mariner Organismen in Form von Mikro- und Nanopartikeln als pharmazeutisch wirksame Mittel sowie als Nahrungsmittelzusätze. Die Verwendung der lipidhaltigen marinen Organismen in Form von Mikro- und Nanopartikeln zur Herstellung von oder Arzneimitteln oder Nahrungsmitteln, auch von diätischen Erzeugnissen ist möglich. Eine Kombination mit anderen Arzneimitteln ist ebenfalls praktikabel.

Die Erfindung kann die Verhinderung der Bindung von nosokomial bedeutsamen Anflugkeimen an Rezeptoren auf der Haut oder Geweben und/oder zur Verhinderung ihres Wachstums auf der Haut oder Geweben verwendet werden. Als besonders wirksam hat sich eine Kombination von Biomassen mariner Organismen mit Vitaminen, insbesondere mit Vitamin C erwiesen.

Die erfindungsgemäße Verwendung schließt auch die Prophylaxe nosokomialer Infektionen ein und eignet sich besonders zur Hemmung von Staphylokokken, insbesondere MRSA (multiresistenter Staphylococcus aureus Stämme), zur Sanierung von mit MRSA kontaminierter Haut.

Ein besonders wirksames Hautpflegemittel wurde dadurch erhalten, dass als Wirkstoffe Xanthonderivate der Formel 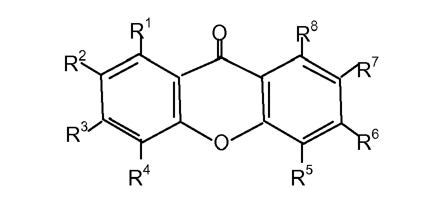 zugegeben werden, wobei R1-R8 in Tabelle 1 aufgeführten Substituenten sein können.

**Tabelle 1:**

| |
|---|
| H, OH, OMe, OAc |
| 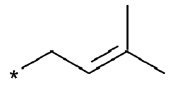 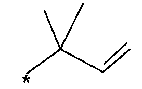 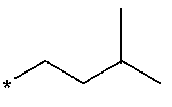 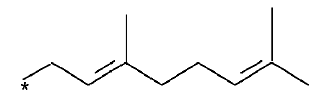 |
| Me, Ac, CH₂OH, CHO, CF₃, COOH, COOMe, CN, CONH₂ |
| Cl, F, NO₂, NH₂, NHAc, NMe₂ |

Als Radikalfänger können zusätzlich Tocopherole und /oder Benzochinone der allgemeinen Formel 2 mit der Kettenlänge n= 1 bis n = 15 enthalten sein, mit den Resten R1-R3, wobei R1, R2 und R3 Wasserstoff, Halogen, Alkyl- oder Alkoxygruppe, R4 als isoprenoide Seitenkette mit 1 bis 10 isoprenoiden Einheiten X oder als aliphatische Seitenkette mit 1 bis 10 CH₃- Gruppen (Formel 3).

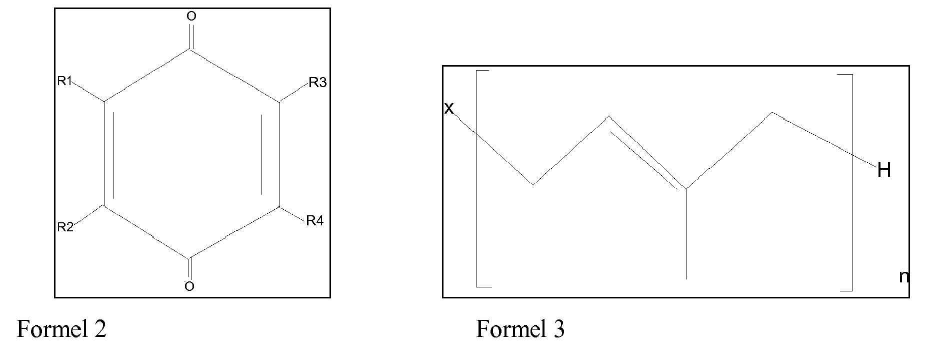

Die erfindungsgemäßen Mittel können Dihydroxybenzaldehyd oder dessen Derivate des mit der allgemeinen Formel 4 enthalten, mit den Resten R1, R2, R3, R4 und R5 als Wasserstoff, Halogen, Alkyl -oder Alkangruppen.

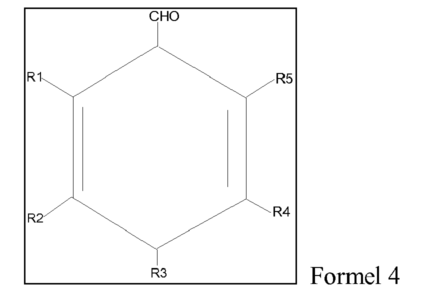

Zahlreiche lipidhaltige marine Organismen enthalten Substanzen, die als unspezifische Immunstimulantien wirken können, d. h. sie stimulieren Leukozyten und wirken als Aktivatoren des retikoloendothelialen Systems. Nach der erfindungsgemäßen Umwandlung der Biomasse dieser Organismen in Mikro- oder Nanopartikel ermöglichen diese Inhaltstoffe weitere Anwendungen. Vorteilhaft ist beispielsweise ein Einsatz in Abdeckmaterialien zur Wundversorgung. Erfindungsgemäß mit Antibiotika dotierte Mikro- und Nanopartikel erlauben eine gesteuerte Freisetzung der antimikrobiellen Wirkstoffe und eine gleichzeitige Immunstimulation. Vorteilhafterweise können diese Mikro- und Nanopartikel zusätzlich mit anorganischen Thiocyanaten oder Hydrothiocyanaten organischer Basen dotiert werden.

Erfindungsgemäß können die Mikro- und Nanopartikel auch zusätzlich DNA enthalten und gestatten somit ihre Verwendung für einen Gentransfer.

Neben der Anwendung in pharmazeutischen Produkten sind auch Anwendungen im diätischen und lebensmitteltechnologischen Bereich erfindungsgemäß. Der große Reichtum lipophiler mariner Organismen an wertvollen Inhaltsstoffen ermöglicht nach erfindungsgemäßer Umwandlung zu Mikro- und Nanopartikel eine gezielte Substituierung von Mangelzuständen. Fette, Vitamine, Mineralstoffe und andere wertvolle Inhaltsstoffe der marinen Organismen können nach Umwandlung in Mikro- und Nanopartikel durch den Säugetierorganismus besser genutzt werden. Eine Anreicherung mit Nahrungsergänzungsstoffen ist bei Anwendung der erfindungsgemäßen Verfahren problemlos möglich. Folgende Gruppen mariner Organismen verfügen über Inhaltsstoffe, die nur in diesen in geeigneter Zusammensetzung und Konzentration vorkommen und die über die erfindungsgemäße Umwandlung in Mikro- und Nanopartikel einer Nutzung zugeführt werden können:
- *Cyanobakterien der Klasse Oscillatoriales, insbesondere die Stämme SPH 03, SPH 04, SPH 05, SPH 06, SPH 09, SPH 10, SPH 11, SPH 12, SPH 13, SPH 14, SPH 20, SPH 21, SPH 22, SPH 23, SPH 25, SPH 26, SPH 29, SPH 32, SPH 34, SPH 37.
- *Cyanobakterien der Klasse Nostocales, insbesondere die Stämme SPH 18, SPH 20, SPH 27, SPH 28, SPH 38
- *Cyanobakterien der Klasse Chroococcales unter besonderer Berücksichtigung der Stämme SPH 07a, SPH 07b, SPH 08, SPH 14, SPH 16, SPH 17, SPH 24, SPH 33, SPH 36, SPH 39, SPH 40, SPH 43 und der Klasse Stigonematales
- *Makroalgen der Gattungen Asparagopsis, Cystoseira, Codium, Dictyota, Dictyopteris, Enteromorpha, Fucus, Gelidium, Gracilaria, Gracilariopsis, Halopteris, Hypoglossum, Laurencia, Plocamium, Polyneura, Sargassum, Solieria, Ulva
- *Thraustochytriden der Gattungen Schizochytrium und Thraustochytrium
- *marine Bakterien der Gattungen Photobacterium, Shewanella und Colwellia.

Besonders vorteilhaft ist, dass die Partikel mittels Rührwerken (Rotor-Stator-Prinzip) und Hochdruckhomogenisation hergestellt werden können, die seit Jahrzehnten zur Herstellung von Fettemulsionen zur parenteralen Ernährung genutzt und daher für eine Produktion von Biomassepartikeln in industriellem Maßstab zur Verfügung stehen. Sie sind von den staatlichen Behörden zur Herstellung von Parenteralia akzeptiert und bedürfen daher keiner neuen aufwendigen Zulassungsverfahren.

Die erfindungsgemäße Umwandlung von Biomassen aus lipidhaltigen marinen Organismen ermöglich darüber hinaus weitere Anwendungen. Die Lipide binden an Kunststoffoberflächen. Das ermöglicht den Einsatz der erfindungsgemäß dotierten Mikro- und Nanopartikel lipidhaltiger mariner Organismen in Slow-release-Systemen zur Prävention implantat-assoziierter Infektionen, vorzugsweise von katheter-assoziierten Infektionen. Die feste Biomatrix ermöglicht den Schutz des inkorporierten Wirkstoffs vor chemischer Zersetzung und eine verzögerte Freigabe des Pharmakons. Durch Wahl geeigneter Tenside und Wirkstoffe ist aber auch eine beschleunigte Freisetzung der Wirkstoffe von der Partikeloberfläche möglich, wenn diese an der Oberfläche gelagert werden, so dass eine gesteuerte Wirkstofffreigabeerfindungsgemäß ist. Das erfindungsgemäße Verfahren ermöglicht die Sterilisation von mit den erfindungsgemäßen Mikro- und Nanopartikeln dotierten Materialien.

Im Gegensatz zu Polymernanopartikeln sind die neuartigen Partikeln aus mariner Biomasse biologisch abbaubar.

Die Merkmale der Erfindung gehen aus den Elementen der Ansprüche und aus der Beschreibung hervor, wobei sowohl einzelne Merkmale als auch mehrere in Form von Kombinationen vorteilhafte Ausführungen darstellen, für die mit dieser Schrift Schutz beantragt wird.

Das Wesen der Erfindung besteht aus einer Kombination aus bekannten (Biomassen aus lipidhaltigen marinen Organismen) und neuen Elementen (der Umwandlung der Biomassen in Mikro- bzw. Nanopartikel), die sich gegenseitig beeinflussen und in ihrer neuen Gesamtwirkung einen Gebrauchsvorteil und den erstrebten Erfolg ergeben, der darin liegt, dass erstmals Wirkstoffträger auf der Basis von lipidhaltigen marinen Mikroorganismen bereitgestellt werden konnten, dass Inhaltsstoffe dieser Organismen bioverfügbar wurden, dass Stoffe, die bislang nicht pharmazeutisch wirksam waren nun neue Eigenschaften haben und dass die Freisetzung der inkorporierten Substanzen durch Wahl der Temperatur, der Wirkstoffe, des Tonmineralanteils und der Tenside gesteuert werden kann.

Die Erfindung soll anhand von Ausführungsbeispielen erläutert werden, ohne auf diese Beispiele beschränkt zu sein.

### Ausführungsbeispiele

### Beispiel 1 :Herstellung von Mikro- und Nanopartikeln aus Biomasse von Cyanobakterien der Ordnung Chrooccocales.

Es wurden Cyanobakterien des Stammes B 30 eingesetzt, der als Eigenisolation aus dem Jasmunder Bodden (Ostsee) gewonnen wurde. Extrakte unterschiedlicher Polarität aus diesem Stamm zeigten in Screening-Test keine antimikrobielle Wirksamkeit.

**Tabelle 2: Rezeptur der Biomasse B 30- Vitamin C - Mikro- und Nanopartikeln**

| **Stoff** | **Menge in g** |
|---|---|
| Biomasse | 5,00 |
| Emulgator (Plantacare 2000) | 0,05 |
| Demineralisiertes Wasser | 45,00 |
| Homogenisationszyklen | 4 |

Die Biomasse wird auf eine Temperatur von 50°C erwärmt. Davon getrennt wird eine wässrige Emulgatorlösung auf die entsprechende Temperatur (50°C) erwärmt. Danach werden beide Phasen bei der gewünschten Homogenisierungstemperatur vereint. Dann wird das Gemisch mit Hilfe eines Ultra Turrax T25 der Fa. Janke und Kunkel GmbH & Co KG (Staufen, Deutschland) in einem Emulgierungsprozess bei 8000 Umdrehungen pro Minute und einer Dauer von 30 Sekunden verarbeitet. Die Suspension wird danach mit einem Kolbenspalt-Hochdruckhomogenisator Micron Lab 40 (APV-Gaulin, Lübeck) bei einem Druck von 500 bar und einer Temperatur von 50°C vier mal homogenisiert.

### Beispiel 2: Herstellung von Mikro- und Nanopartikeln mit Vitamin C aus Biomasse von Cyanobakterien der Ordnung Chrooccocales.

Es wurden Cyanobakterien des Stammes B 30 eingesetzt, der als Eigenisolation aus dem Jasmunder Bodden(Ostsee) gewonnen wurde. Extrakte unterschiedlicher Polarität aus diesem Stamm zeigten in Screening-Test keine antimikrobielle Wirksamkeit.

**Tabelle 3: Rezeptur der Biomasse B 30- Vitamin C - Mikro- und Nanopartikeln**

| **Stoff** | **Menge in g** |
|---|---|
| Biomasse | 5,00 |
| Emulgator (Plantacare 2000) | 0,05 |
| Demineralisiertes Wasser | 45,00 |
| Vitamin C | 5 |
| Homogenisationszyklen | 4 |

Anschließend wurden in die Biomasse 5 g Vitamin C eingearbeitet. Davon getrennt wird eine wässrige Emulgatorlösung auf die entsprechende Temperatur (50°C) erwärmt. Danach werden beide Phasen bei der gewünschten Homogenisierungstemperatur vereint. Das Gemisch wird mit Hilfe eines Ultra Turrax T25 der Fa. Janke und Kunkel GmbH & Co KG (Staufen, Deutschland) in einem Emulgierungsprozess bei 8000 Umdrehungen pro Minute und einer Dauer von 30 Sekunden verarbeitet. Die Suspension wird danach mit einem Kolbenspalt-Hochdruckhomogenisator Micron Lab 40 (APV-Gaulin, Lübeck) bei einem Druck von 500 bar und einer Temperatur von 50°C vier mal homogenisiert.

Die beiden Mikro- und Nanopartikeln B30 und Vitamin C wurden miteinander gemischt.

### Beispiel 3: Herstellung von Mikro- und Nanopartikeln aus Biomasse von Cyanobakterien der Gattung Spirulina.

Es wurden Cyanobakterien der kommerziell verfügbaren Gattung Spirulina eingesetzt.

**Tabelle 4: Rezeptur der Spirulina - Mikro- und Nanopartikeln**

| **Stoff** | **Menge in g** |
|---|---|
| Biomasse | 5,00 |
| Emulgator (Plantacare 2000) | 0,05 |
| Demineralisiertes Wasser | 45,00 |
| Homogenisationszyklen | 4 |

Die Biomasse wird bei einer Temperatur von 25°C in eine wässrige Emulgatorlösung dispergiert. Anschließend wird das Gemisch mit Hilfe eines Ultra Turrax T25 der Fa. Janke und Kunkel GmbH & Co KG (Staufen, Deutschland) in einem Emulgierungsprozess bei 8000 Umdrehungen pro Minute und einer Dauer von 30 Sekunden verarbeitet. Die Suspension wird danach mit einem Kolbenspalt - Hochdruckhomogenisator Micron Lab 40 (APV-Gaulin, Lübeck) bei einem Druck von 500 bar und einer Temperatur von 50°C vier mal homogenisiert.

### Beispiel 4: Herstellung von Mikro- und Nanopartikeln aus Biomasse von Cyanobakterien der Gattung Oscillatoria

Es wurden Cyanobakterien die Art Oscillatoria redekei HUB 051 eingesetzt.

**Tabelle 5: Rezeptur der Oscillatoria redekei HUB 051 - Partikel**

| **Stoff** | **Menge in g** |
|---|---|
| Biomasse | 5,00 |
| Emulgator (Plantacare 2000) | 0,05 |
| Demineralisiertes Wasser | 45,00 |
| Homogenisationszyklen | 4 |

Die Biomasse wird bei einer Temperatur von 25°C in eine wässrige Emulgatorlösung dispergiert. Anschließend wird das Gemisch mit Hilfe eines Ultra Turrax T25 der Fa. Janke und Kunkel GmbH & Co KG (Staufen, Deutschland) in einem Emulgierungsprozess bei 8000 Umdrehungen pro Minute und einer Dauer von 30 Sekunden verarbeitet. Die Suspension wird danach mit einem Kolbenspalt-Hochdruckhomogenisator Micron Lab 40 (APV-Gaulin, Lübeck) bei einem Druck von 500 bar und einer Temperatur von 50°C vier mal homogenisiert.

### Beispiel 5: Herstellung von Mikro- und Nanopartikeln aus Biomasse von Cyanobakterien der Ordnung Nostocales

Es wurden Cyanobakterien der Ordnung Nostocales eingesetzt.

**Tabelle 6 : Rezeptur der Nostocales - Mikro- und Nanopartikeln**

| **Stoff** | **Menge in g** |
|---|---|
| Biomasse | 5,00 |
| Emulgator (Plantacare 2000) | 0,05 |
| Demineralisiertes Wasser | 45,00 |
| Homogenisationszyklen | 4 |

Die Biomasse wird bei einer Temperatur von 25°C in eine wässrige Emulgatorlösung dispergiert. Anschließend wird das Gemisch mit Hilfe eines Ultra Turrax T25 der Fa. Janke und Kunkel GmbH & Co KG (Staufen, Deutschland) in einem Emulgierungsprozess bei 8000 Umdrehungen pro Minute und einer Dauer von 30 Sekunden verarbeitet. Die Suspension wird danach mit einem Kolbenspalt-Hochdruckhomogenisator Micron Lab 40 (APV-Gaulin, Lübeck) bei einem Druck von 500 bar und einer Temperatur von 50°C vier mal homogenisiert.

### Beispiel 6: Herstellung von Mikro- und Nanopartikeln aus Biomasse von Cyanobakterien der Ordnung Chrooccocales mit Vitamin C nach dem Lösungsmittelverfahren

Als Biomasse wurde der im Beispiel 1 beschriebene Stamm B 30 eingesetzt.

**Tabelle 7: (Lösungsmittelverfahren)**

| | |
|---|---|
| Wasser | 45,00 g gereinigtes, filtriertes Wasser |
| organisches Lösungsmittel | 25 ml n-Hexan |
| Biomasse | 0,5 g Chrooccocales-Cyanobakterien |
| Vitamin | 5 g Vitamin C |
| Tensid | 0,5 g Plantacare |

Zunächst wird die Biomasse in n-Hexan gelöst, wobei die Lipide aus der Biomasse gelöst werden. Die Biomasse wird ca. 3 Stunden in dem Lösungsmittel gerührt. Anschließend wird das Lösungsmittel mittels eines Rotationsverdampfer abgezogen. Dabei bildet sich eine Lipidschicht an der Kolbenoberfläche. Zur Redispergierung wird eine Vitamin C-haltige Lösung benötigt: Vitamin C wird in eine Plantacare-Lösung gebracht und nach einem 90 sekundigen Dispergiervorgang 4 mal homogenisiert. Die entstehende Vitamin C-haltige Lösung wird in den Kolben mit der Lipidschicht gebracht. Die Lipidschicht löst sich bei der anschließenden Rotation des Kolbens und bildet dadurch verkapselte Mikro- und Nanopartikeln. Die Rotation dauert zirka 10 Stunden bei einer Temperatur von 40 ° C.

### Beispiel 7: Herstellung von Mikro- und Nanopartikeln aus Biomasse-Norlichexanthon

**Tabelle 8: Rezeptur der Biomasse- Norlichexanthon - Mikro- und Nanopartikeln**

| **Stoff** | **Menge in g** |
|---|---|
| Wirkstoff (Norlichexanthon) | 0,05 |
| Biomasse (Cyanobakterien- Spirulina) | 5,00 |
| Emulgator (Plantacare 2000) | 0,05 |
| Demineralisiertes Wasser | 45,00 |
| Homogenisationszyklen | 4 |

Die Biomasse wird auf eine Temperatur von 50°C erwärmt und anschließend der verwendete marine Wirkstoff Norlichexanthon darin dispergiert bzw. gelöst. Davon getrennt wird eine wässrige Emulgatorlösung auf die entsprechende Temperatur (50°C) erwärmt. Danach werden beide Phasen bei der gewünschten Homogenisierungstemperatur vereint. Anschließend wird das Gemisch mit Hilfe eines Ultra Turrax T25 der Fa. Janke und Kunkel GmbH & Co KG (Staufen, Deutschland) in einem Emulgierungsprozess bei 8000 Umdrehungen pro Minute und einer Dauer von 30 Sekunden verarbeitet. Die Suspension wird danach mit einem Kolbenspalt-Hochdruckhomogenisator Micron Lab 40 (APV-Gaulin, Lübeck) bei einem Druck von 500 bar und einer Temperatur von 50°C vier mal homogenisiert.

### Beispiel 8: Herstellung Mikro- und Nanopartikeln aus Biomasse - Vitamin E

**Tabelle 9: Rezeptur der Biomasse- Vitamin E- Mikro- und Nanopartikeln**

| **Stoff** | **Menge in g** |
|---|---|
| Wirkstoff Vitamin E | 0,05 |
| Biomasse (Cyanobakterien Spirulina) | 5,00 |
| Emulgator (Plantacare 2000) | 0,05 |
| Demineralisiertes Wasser | 45,00 |
| Homogenisationszyklen | 4 |

In die Biomasse wird das Vitamin E dispergiert. Davon getrennt wird eine wässrige Emulgatorlösung hergestellt. Anschließend wird das Gemisch mit Hilfe eines Ultra Turrax T25 der Fa. Janke und Kunkel GmbH & Co KG (Staufen, Deutschland) in einem Emulgierungsprozess bei 8000 Umdrehungen pro Minute und einer Dauer von 30 Sekunden verarbeitet. Die Suspension wird danach mit einem Kolbenspalt-Hochdruckhomogenisator Micron Lab 40 (APV-Gaulin, Lübeck) bei einem Druck von 500 bar und einer Temperatur von 50°C vier mal **homogenisiert.**

### Beispiel 9: Herstellung Mikro- und Nanopartikeln aus Biomasse - Provitamin Q10

**Tabelle 10 Rezeptur der Biomasse-Provitamin Q10- Mikro- und Nanopartikeln**

| **Stoff** | **Menge in g** |
|---|---|
| Wirkstoff Provitamin Q10_ | 0,05 |
| Biomasse (Cyanobakterien -Spirulina) | 5,00 |
| Emulgator (Plantacare 2000) | 0,05 |
| Demineralisiertes Wasser | 45,00 |
| Homogenisationszyklen | 4 |

In die Biomasse wird das Provitamin Q10_dispergiert. Davon getrennt wird eine wässrige Emulgatorlösung hergestellt. Anschließend wird das Gemisch mit Hilfe eines Ultra Turrax T25 der Fa. Janke und Kunkel GmbH & Co KG (Staufen, Deutschland) in einem Emulgierungsprozess bei 8000 Umdrehungen pro Minute und einer Dauer von 30 Sekunden verarbeitet. Die Suspension wird danach mit einem Kolbenspalt-Hochdruckhomogenisator Micron Lab 40 (APV-Gaulin, Lübeck) bei einem Druck von 500 bar und einer Temperatur von 50°C vier mal homogenisiert.

### Beispiel 10: Testung von Mikro- und Nanopartikeln "Biomasse B30/Vitamin C" hergestellt nach Beispiel 1 und 2 am Tiermodell (Kuheuterzitze).

Die beiden Substanzen wurden 1:1 miteinander gemischt. Methodik:

Eine Stunde nach Tötung der Kuh wurden die Euterzitzen verarbeitet. Die Zitzen wurden von der Fettschicht befreit. Danach wurden die Zitzen auf Metallstäbe entsprechender Größe gesteckt und mit Schellen befestigt. Die Zitzen wurden mit 70%igem Alkohol abgerieben. 20 µl Testsubstanz wurde aufpipettiert und mit einem Glasspatel verrieben, und bei Raumtemperatur 30-45 Minuten getrocknet. Die Kontamination erfolgte mit 10 µl des Norddeutschen Stammes, MF 0,5 1:10 verdünnt. Nach Bebrütung bei 30°C für 1,5 Stunden wurden die Hautareale auf Müller-Hinton Platten ausgestrichen und bei 37°C inkubiert.

### Auswertung:

Bei den Kontrollen wurden ausschließlich Keimzahlen > 100 gefunden. Daraus wurden die mittlere Keimzahl n und die Streuung berechnet.

Bei der Untersuchung der Zubereitungen wurden Keimzahlen zwischen 0 und 100 gefunden. Daraus wurde die mittlere Keimzahl m auf der Basis der Poisson-Verteilung nach folgender Formel berechnet: m = ln (Zahl der Proben ohne Keimnachweis/Gesamtzahl der Proben).

### Statistische Auswertung:

Die Anzahl der Hautareale mit und ohne Keimnachweis für die Kontrolle ohne Behandlung und nach Behandlung mit dem Testprodukt wurde mit Hilfe des Chi-Quadrat-Testes auf Signifikanz geprüft.

### Ergebnisse

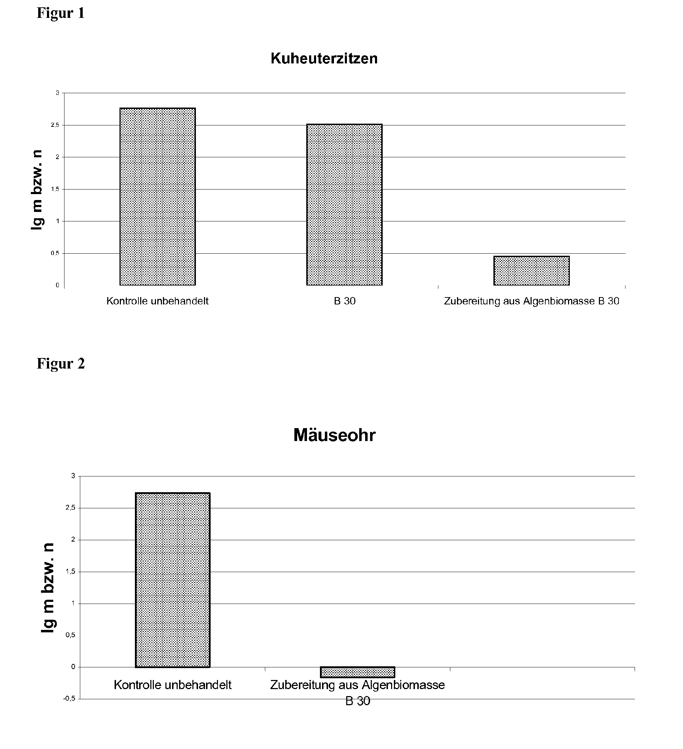

Die Testung der Mikro- und Nanopartikeln, herstellt nach Beispiel 1) bewirkte am Hautmodell (Kuheuterzitze) eine ausgeprägte Reduktion der übertragen Kontamination mit MRSA. Die Zahl der Wiederholungen am Kuheuterzitze betrug für die unbehandelten Kontrollen 163. Bei allen 163 Proben war S. aureus nachweisbar. Die mittlere Keimzahl betrug 759. Auch bei allen Proben, die mit Wollwachsalkoholsalbe behandelt waren, wurde S. aureus nachgewiesen. Nach Anwendung des Testprodukten waren 11 Hautareale mit Keimnachweis und 11 ohne Keimnachweis. Daraus errechnet sich eine im Mittel zu erwartende Keimzahl von 0,7 (Figur 1). Die Keimzahlreduktion ist hoch signifikant.

### Beispiel 11 Testung von Mikro- und Nanopartikeln "Biomasse B30/Vitamin C" hergestellt nach Beispiel 1 und 2 am Tiermodell Mäuseohr

### Methodik

Als Testmodell wurden Mäuseohren verwendet. Die Donator-Tiere als Infektionsquelle blieben unbehandelt. Akzeptortiere wurde 3 Tage einmal täglich mit "Biomasse B30/Vitamin C" hergestellt nach Beispiel 1 behandelt.Dazu wurden 10 µl Testsubstanz wurde aufpipettiert und mit einem Glasspatel verrieben, und bei Raumtemperatur getrocknet. Die Tiere wurden nach vier Tagen getötet. Die Kontamination der Donatortiere erfolgte mit 5 µl des Norddeutschen Stammes, MF 0,5 1:10 verdünnt. Dazu wurde ein unbehandeltes Ohr kontaminiert und danach 90 Minuten bei 30 °C bebrütet. Die mit Biomasse behandelten Ohren wurden auf entsprechende Stempel aufgezogen. Mit Druck wurden die kontaminierten Ohren auf die auf die unbehandelten Ohren 10 Sekunden gepresst.

Nach Bebrütung bei 30°C für 1,5 Stunden wurden die Hautareale der Akzeptor-Ohren auf Müller-Hinton Platten ausgestrichen und bei 37°C inkubiert. Auswertung:

Die Auswertung und statistische Sicherung erfolgte wie im Beispiel 9 beschrieben. Ergebnisse:

Die Testung der Mikro- und Nanopartikeln, hergestellt nach Beispiel 1 und 2, zeigte auch im Donator-Akzeptor-Versuch, mit dem die Infektionsübertragung mittels Hautkontakt simuliert wurde , eine signifikante Reduktion der übertragenen Keime.

Die Zahl der Wiederholungen am Mäuseohr betrug für die unbehandelten Kontrollen 163, dabei wurde auf Hautarealen Keimwachstum nachgewiesen. Bei Vorbehandlung mit der Zubereitung nach Beispiel 1 und 2 wurden 12 Hautareale mit und 5 ohne Keimnachweis. Daraus ergibt sich, das nach einer entsprechenden Vorbehandlung im Mittel nur noch 1,2 Keime/cm² zu erwarten sind (Figur 2).

### Beispiel 12 Testung von Mikro- und Nanopartikeln "Biomasse B30/Vitamin C" hergestellt nach Beispiel 1 und 2 am Hängebauchschwein

### Methodik:

Für die Testung stand ein Hängebauchschwein zur Verfügung. 2 Stunden nach Tötung des Tieres wurde von der Bauchunterseiten, in Nähe der Zitzen Haut herausgeschnitten, die Fettschicht weitestgehend entfernt und in Stücke geschnitten. Diese wurde auf Vorrichtungen gespannt, so dass ca. 1cm² zur Bearbeitung zur Verfügung stand. Diese Hautflächen wurden rasiert bzw. die Borsten mit der Schere abgeschnitten und anschließend 2 x mit 70% Ethanol abgerieben. 20 µl Testsubstanz wurden aufgebracht und 45 min inkubiert. Die Kontamination erfolgte mit 10 µl des Norddeutschen Stammes, MF 0,5 1:10 verdünnt. Nach Bebrütung bei 30°C für 1,5 Stunden wurden die Hautareale auf Müller-Hinton Platten ausgestrichen und bei 37°C inkubiert. Nach der Bebrütung erfolgte die Auszählung der Keime..

### Ergebnis

Trotz der vorgenommenen Hautdesinfektion waren auf dem Abstrich zahlreiche koagulasenegative apathogene Staphylokokken der normalen Hautflora des Schweins nachweisbar. Die koagulase positiven Staphyloccocus aureus-Stämme, mit denen die Kontamination vorgenommen worden war, waren bei der unbehandelten Kontrolle zahlreich neben S. epidermidis nachweisbar, bei der erfindungsgemäß behandelten Tieren waren alle untersuchten Kolonien koagulase negativ.

### Beispiel 13 Testung von Mikro- und Nanopartikeln "Biomasse B30/Vitamin C" hergestellt nach Beispiel 6 am Hautmodell nach Beispiel 10

### Ergebnis

Die Zahl der Wiederholungen an der Kuheuterzitze betrug für die unbehandelten Kontrollen 158. Die Zahl der Hautareale mit Keimnachweis betrug nach Anwendung der Mikro-und Nanopartikel 2 und ohne Keimnachweis 4. Das bedeutet, dass nach Behandlung mit der erfindungsgemäßen Zubereitung nach Beispiel 5 im Mittel nur noch 0,4 Keime/cm² zu erwarten sind. Die Keimzahlreduktion ist im Chi-Quadrat-Test hoch signifikant.

### Beispiel 14 Testung von Mikro- und Nanopartikeln "Biomasse B30/Vitamin C" hergestellt nach Beispiel 6 am Hautmodell nach Beispiel 11

### Ergebnisse:

Die Testung der Mikro- und Nanopartikeln, hergestellt nach Beispiel 6, zeigte im Donator-Akzeptor-Versuch, mit dem die Infektionsübertragung mittels Hautkontakt simuliert wurde , eine signifikante Reduktion der übertragenen Keime. Die Zahl der Hautareale mit Keimnachweis betrug 1 und ohne Keimnachweis 5. Daraus ergibt sich, dass nach der erfindungsgemäßen Vorbehandlung im Mittel nur noch 0,18 Keime beobachtet werden. Die Reduktion der übertragenen Keime ist im Chi-Quadrat-Test hoch signifikant.

### Beispiel 15: Prüfung von Mikro- und Nanopartikeln, hergestellt nach den Beispielen am Hautmodell nach Beispiel 10

### Ergebnisse

Die Mikro- und Nanopartikeln Norlichexathon-Vitamin E-Q10 (Lösungsmittelverfahren) wurden nach Beispiel 7, 8, 9 hergestellt und im Verhältnis 1 : 1 : 1 gemischt. Die Testung erfolgte nach dem in Beispiel 10 dargestellten Verfahren.

### Ergebnis

Die Anzahl der getesteten Zubereitungen mit der Norlichexanthon betrug 22. Die Zahl der Hautareale mit Keimnachweis betrug 19 und ohne Keimnachweis 3. Im Mittel sind nach Vorbehandlung mit erfindungsgemäßen Zubereitungen nach Beispiel 8 noch 2 Keime zu erwarten.

### Beispiel 16 Herstellung von Ubichinon Q1 -Biomasse - Partikeln

**Tabelle 10 Rezeptur der Ubichinon - Algenbiomasse - Partikeln**

| **Stoff** | **Menge in g** |
|---|---|
| Wirkstoff (Ubichinon Q1) | 0,05 |
| Biomasse Ocillatoria redikei HUB051 | 5,00 |
| Emulgator (Plantacare 2000) | 0,05 |
| Demineralisiertes Wasser | 45,00 |
| Homogenisationszyklen | 4 |

Die Biomasse wird auf eine Temperatur von 50 °C erwärmt und anschließend Ubichinon Q1 darin dispergiert bzw. gelöst. Davon getrennt wird eine wässrige Emulgatorlösung auf die entsprechende Temperatur (50 °C) erwärmt. Danach werden beide Phasen bei der gewünschten Homogenisierungstemperatur vereint. Anschließend wird das Gemisch mit Hilfe eines Ultra Turrax T25 der Fa. Janke und Kunkel GmbH & Co KG (Staufen, Deutschland) in einem Emulgierungsprozess bei 8000 Umdrehungen pro Minute und einer Dauer von 30 Sekunden verarbeitet.

Die Suspension wird danach mit einem Kolbenspalt-Hochdruckhomogenisator Micron Lab 40 (APV-Gaulin, Lübeck) bei einem Druck von 500 bar und einer Temperatur von 50 °C vier mal homogenisiert.

### Beispiel 17: Verhinderung der Übertragung von MRSA bei Hautkontakten mit der Zubereitung nach Beispiel 15

### Methodik

Für die Versuche wurden Schwänze von Mäusen aus keimfreier Haltung verwendet. Um jede Fremdkontamination auszuschließen, wurde sie vor Versuchsbeginn für 5 min in 70 %igen Alkohol eingelegt und danach unter der Laminarbox getrocknet.

Die Donatormäuseschwänze als Infektionsquelle wurden durch Einlegen (30 s bis 4 min) in eine verdünnte MRSA-Kultur (Norddeutscher Stamm, MF-Standard 0,5 bzw. 0,3) kontaminiert und danach 24 h bei 37 °C bebrütet. Bei Abstrichen von diesen Donatoren wurden Keimzahlen > 100.000 nachgewiesen.

In die Mäuseschwänze, die den empfänglichen Organismus simulieren (Akzeptoren) wurden die Versuchssubstanzen zweimal täglich einmassiert.

Die Infektionsübertragung vom Donator zum Akzeptor erfolgte durch Hautkontakt mit den Donatoren für 30 s bis 1 min auf der Schüttelmaschine bei 600 U/min. 2 h bzw. 24 h nach der Kontamination wurden die Donatoren auf Blut-Müller-Hinton-Agarplatten ausgestrichen. Nach 24 h Bebrütung der Agarplatten bei 37 °C wurden die Kolonien ausgezählt.

### Ergebnisse:

**Tabelle 12**

| Donator | | Hautkontakt (min) | Vorbehand- lung des Akzeptors | Keimzahl des Akzeptors | |
|---|---|---|---|---|---|
| McFarland-. Standard | Kontamination (min) | | | 2 h | 24 h |
| 0,5 | 5 | 0,5 1 | ohne | >10000 > 10000 | >10000 > 10000 |
| 0,5 | 5 | 0,5 1 | 2 d Zubereitung nach Beispiel 15 | 143 | 2 |
| | | | | 80 | 36 |
| 0,5 | 3 | 0,5 | ohne | >10000 | >10000 |
| | | | 2 d Zubereitung nach Beispiel 15 | | 6 |
| 0,3 | 1 | 1 | ohne | | > 10000 |
| | | | 2 d Zubereitung nach Beispiel 15 | | 2 |

Tabelle 12 zeigt Keimzahlen auf Mäuseschwänzen (Akzeptoren) in Abhängigkeit von der Vorbehandlung nach Kontakt mit MRSA kolonisierten Mäuseschwänzen (Donatoren).

Beim Ausstreichen der Akzeptoren unmittelbar nach dem Hautkontakt sind die Platten vollständig bewachsen (Keimzahl > 10 000). Das gilt gleicherweise für vorbehandelte wie für Kontrollen ohne Vorbehandlung. Das bedeutet, das eine massive Übertragung der MRSA in diesem Modell gut simuliert werden kann. Bei Versuchs- und Kontrollgruppe wird die gleiche Keimzahl übertragen.

2 h bzw.24 h sind dagegen nur noch geringen Keimzahlen bei den vorbehandelten Akzeptoren nachzuweisen, während die Keimzahlen bei den unbehandelten Kontrollen unverändert hoch sind. (Tab. 12). Auch mit diesem Versuchsmodell kann also die Unterbrechung der Infektionswege durch eine Vorbehandlung mit den erfindungsgemäßen Formulierungen nachgewiesen werden.

### Beispiel 18: Teilchengrößenbestimmung der Mikro- und Nanopartikeln, hergestellt nach Beispiel 3

### Methodik

Die Teilchengröße durch Photonenkorrelationsspektroskopie wurde mit Hilfe eines Malvernzizer III(Malvern, UK) bestimmt.

### Beispiel 19: Herstellung von Mikro- und Nanopartikeln mit DNA aus Biomasse von Cyanobakterien der Ordnung Chrooccocales.

**Tabelle 13: Rezepturbeispiele Einarbeitung der DNA in Algenbiomasse**

| **Stoff** | **Rezeptur 1** | **Rezeptur 2** | **Rezeptur 3** |
|---|---|---|---|
| Wirkstoff (Heringssper-mien -DNA) | 5 %_ | 10 %_ | 15 % |
| Algenbiomassegrundlage der Ordnung Chroocco-cales | 5,00 g Cyanobakterien | | |
| Emulgator | 0,60 g Plantacare® | 1,25 g Pluronic® F-68 | 0,60 g Miranol Ultra 32 |
| Demineralisiertes Wasser | 45,00 g | | |
| Homogenisationszyklen | 4 | 3 | 3 |

Bei der Inkorporation der DNA (Sigma-Aldrich, Deisenhofen (Deutschland)) in Algenbiomasse-Partikeln werden die Herstellungsparameter leicht variiert, um einer Zerstörung der DNS entgegenzuwirken:

| | |
|---|---|
| - Vorhomogenisation mit dem Ultra-Turrax: | 30 sec |
| - Homogenisationstemperatur: | 55°C |
| - Homogenisationsdruck: | 500 bar |
| - Zahl der Homogenisationszyklen: | 2 |

Dabei wird die DNA einmal in getrockneter Form nach Lyophilisation in der Algenbiomasse dispergiert und homogenisiert. Zum anderen wird die in Wasser gelöste DNS einfach zur wässrigen Emulgatorlösung hinzugegeben und mit dieser zusammen in der erwärmten Algenbiomasse verteilt, woran sich dann ebenfalls die Homogenisation anschließt.

### Beispiel 20 Herstellung von Mikro- und Nanopartikeln mit Trimacinolon aus Biomasse von Cyanobakterien der Ordnung Chrooccocales

**Tabelle 14: (Lösungsmittel-Emulsionsverfahren)**

| | |
|---|---|
| Wasser | 45,00 g gereinigtes, filtriertes Wasser |
| organisches Lösungsmittel | 7,50 g Dichlormethan |
| Algenbiomasse (Spirulina) | 1,12 g Cyanobakterien (15 % bezogen auf Dichlormethan) |
| Triamcinolon | 0,12 g Triamcinolon |
| lipophiler Emulgator | 0,38 g Epikuron® 170 (5 % bezogen auf Dichlormethan) |
| Cotensid | 0,11 g Pluronic® F-68 (1,5 % bezogen auf Dichlormethan) |

Die Herstellung der Trimacinolon-Cyanobakterien-Mikropartikeln basiert auf der Bereitung einer Emulsion aus Wasser und einer Lösung der Cyanobakterien mit dem Wirkstoff Triamcinolon in dem Lösungsmittel Dichlormethan. Nach einem Homogenisationsschritt wird das organische Lösungsmittel (Dichlormethan) durch Verdampfen entfernt, wobei die Algenbiomasse in Form von festen Nanopartikeln ausfallt. Die Stabilisierung der Emulsion bzw. Dispersion erfolgt durch ein geeignetes Tensidgemisch (Pluronic@ F-68).

Als Geräte werden ein Ultra- Turrax T25 der Firma Janke & Kunkel GmbH & Co KG (Staufen/ Deutschland), sowie ein Kolben-Spalt-Homogenisator Micron Lab 40 der Firma APV Gaulin (Lübeck Deutschland) mit 7 Zyklen und bei 800 bar eingesetzt. Zusätzlich wird ein Rotationsverdampfer Rotavapor R114 mit einem angeschlossenen Vakuumsystem B178 der Finna Büchi (Flawil/ Schweiz) bei einem Druck von 0,7 bar 60 min lang verwendet.

### Beispiel 21: Herstellung von Ubichinon Q1 - Algenbiomasse - Partikeln

**Tabelle 15: Rezeptur der Ubichinon - Algenbiomasse - Partikel**

| **Stoff** | **Menge in g** |
|---|---|
| Wirkstoff (Ubichinon Q1) | 0,05 |
| Algenbiomasse | 5,00 |
| Emulgator (Plantacare 2000) | 0,05 |
| Demineralisiertes Wasser | 45,00 |
| Homogenisationszyklen | 4 |

Die Algenbiomasse wird auf eine Temperatur von 50°C erwärmt und anschließend der verwendete Wirkstoff Ubichinon Q1 darin dispergiert bzw. gelöst. Davon getrennt wird eine wässrige Emulgatorlösung auf die entsprechende Temperatur (50°C) erwärmt. Danach werden beide Phasen bei der gewünschten Homogenisierungstemperatur vereint. Anschließend wird das Gemisch mit Hilfe eines Ultra Turrax T25 der Fa. Janke und Kunkel GmbH & Co KG (Staufen, Deutschland) in einem Emulgierungsprozess bei 8000 Umdrehungen pro Minute und einer Dauer von 30 Sekunden verarbeitet. Die Suspension wird danach mit einem Kolbenspalt-Hochdruckhomogenisator Micron Lab 40 (APV-Gaulin, Lübeck) bei einem Druck von 500 bar und einer Temperatur von 50°C vier mal homogenisiert.

### Beispiel 22: Tabelle 16: Rezepturbeispiele Einarbeitung von DNA in Algenbiomasse

| **Stoff** | **Rezeptur 1** | **Rezeptur 2** | **Rezeptur 3** |
|---|---|---|---|
| Wirkstoff (DNA) | 5 %_ | 10 %_ | 15 % |
| Algenbiomassegrundlage | 5,00 g Cyanobakterien | | |
| Emulgator | 0,60 g Plantacare® | 1,25 g Pluronic®F- 68 | 0,60 g Miranol Ultra 32 |
| Demineralisiertes Wasser | 45,00 g | | |
| Homogenisationszyklen | 4 | 3 | 3 |

Bei der Inkorporation der DNS in Algenbiomasse-Partikeln werden die Herstellungsparameter leicht variiert, um einer Zerstörung der DNS entgegenzuwirken:

| | |
|---|---|
| - Vorhomogenisation mit dem Ultra-Turrax: | 30 sec |
| - Homogenisationstemperatur: | 55°C |
| - Homogenisationsdruck: | 500 bar |
| - Zahl der Homogenisationszyklen: | 2 |

Dabei wird die DNS einmal in getrockneter Form nach Lyophilisation in der Algenbiomasse dispergiert und homogenisiert. Zum anderen wird die in Wasser gelöste DNS einfach zur wässrigen Emulgatorlösung hinzugegeben und mit dieser zusammen in der erwärmten Algenbiomasse verteilt, woran sich dann ebenfalls die Homogenisation anschließt.

### Beispiel 23: Herstellung von Trimacinolon-Algebiomasse-Mikropartikeln

**Tabelle 17: (Lösungsmittel-Emulsionsverfahren)**

| | |
|---|---|
| Wasser | 45,00 g gereinigtes, filtriertes Wasser |
| organisches Lösungsmittel | 7,50 g Dichlormethan |
| Algenbiomasse | 1,12 g Cyanobakterien (15 % bezogen auf Dichlormethan) |
| Triamcinolon | 0,12 g Triamcinolon |
| lipophiler Emulgator | 0,38 g Epikuron® 170 (5 % bezogen auf Dichlormethan) |
| Cotensid | 0,11 g Pluronic® F-68 (1,5 % bezogen auf Dichlormethan) |

Die Herstellung der Trimacinolon-Cyanobakterien-Mikropartikeln basiert auf der Bereitung einer Emulsion aus Wasser und einer Lösung der Cyanobakterien mit dem Wirkstoff Triamcinolon in dem Lösungsmittel Dichlormethan. Nach einem Homogenisationsschritt wird das organische Lösungsmittel (Dichlormethan) durch Verdampfen entfernt, wobei die Algenbiomasse in Form von festen Nanopartikeln ausfallt. Die Stabilisierung der Emulsion bzw. Dispersion erfolgt durch ein geeignetes Tensidgemisch (Pluronic@ F-68).

Als Geräte werden ein Ultra- Turrax T25 der Firma Janke & Kunkel GmbH & Co KG (Staufen/ Deutschland), sowie ein Kolben-Spalt-Homogenisator Micron Lab 40 der Firma APV Gaulin (Lübeck Deutschland) mit 7 Zyklen und bei 800 bar eingesetzt. Zusätzlich wird ein Rotationsverdampfer Rotavapor R114 mit einem angeschlossenen Vakuumsystem B178 der Finna Büchi (Flawil/ Schweiz) bei einem Druck von 0,7 bar 60 min lang verwendet.

### Beispiel 24: Einfluss von Mikro- und Nanopartikeln mit Aggregaten aus lipidhaltigen marinen Organismen und Tonmineralien auf das Wachstum von Amnion-Epithelzellen.

Cyanophycea wurden bis zu 2 Monaten mit Zusätzen von Bentonit, Friedländer Ton und Kaolin kultiviert. Bereits nach kurzer Inkubationszeit bilden sich Aggregate, während bei den Kontrollen ohne mineralische Zusätze diese erst nach 2 Monaten und in geringerem Umfang gebildet werden. Das Mineral mit der höchsten Absorptionsfähigkeit (Bentonit) zeigt eine geringere Tendenz zur Bildung der Aggregate als Kaolin mit einer geringen Absorptionsfähigkeit. Durch die Wahl der mineralischen Komponenten kann die Aggregatbildung und die Absorptionsfähigkeit der gebildeten Aggregate gesteuert werden. Die Aggregate wurden anschließend mit Hilfe eines Ultra Turrax T25 der Fa. Janke und Kunkel GmbH & Co KG (Staufen, Deutschland) in einem Emulgierungsprozess bei 8000 Umdrehungen pro Minute und einer Dauer von 30 Sekunden verarbeitet.

Bei einer Einwirkung auf FL-Zellen verstärken insbesondere Aggregate mit Bentonit das Zellwachstum signifikant (p = <0,001). Die Beeinflussung ist deutlich stärker als die Wachstumsförderung durch Friedländer Ton (p = 0,024). Der Einfluss von Kaolin ist dagegen nicht signifikant (p =0,2786).

### Beispiel 25: Ermittlung der antibakteriellen Wirksamkeit im Agardiffusionshemmtest.

**Tabelle 18:**

| Erreger | Trihydroxy- benzaldehyd | Trihydroxy-benzaldehyd 3 %ig in dickflüssigen Paraffin | Trihydroxy-benzaldehyd 10 %ig in dickflüssigen Paraffin |
|---|---|---|---|
| Norddeutscher Stamm | | 12 mm | 14 mm |

### Beispiel 26: Dekontamination von mit MRSA besiedelter Haut im Mauseschwänzchentest

**Tabelle 19: Keimzahlen auf Mäuseschwänzen (Akzeptoren) in Abhängigkeit von der Vorbehandlung nach Kontakt mit MRSA kolonisierten Mäuseschwänzen (Donatoren).**

| Donator | | Hautkontakt (min) | Vorbehand-lung des Akzeptors | Keimzahl des Akzeptors | |
|---|---|---|---|---|---|
| McFarland-. Standard | Kontamination (min) | | | 2 h | 24 h |
| 0,5 | 5 | 0,5 1 | ohne | >10000 > 10000 | >10000 > 10000 |
| 0,5 | 5 | 0,5 1 | 2 d Gemisch | 143 | 2 |
| | | | | 80 | 36 |
| 0,5 | 3 | 0,5 | ohne | >10000 | >10000 |
| | | | 2 d Gemisch | | 6 |
| 0,3 | 1 | 1 | ohne | | > 10000 |
| | | | | | 2 |

### Beispiel 27: Verhinderung der Übertragung von MRSA bei Hautkontakten

### Methodik:

Für die Versuche wurden Schwänze von Mäusen aus keimfreier Haltung verwendet. Um jede Fremdkontamination auszuschließen, wurde sie vor Versuchsbeginn für 5 min in 70 %igen Alkohol eingelegt und danach unter der Laminarbox getrocknet.

Die Donatormäuseschwänze wurden durch Einlegen (30 s bis 4 min) in eine verdünnte MRSA-Kultur (Norddeutscher Stamm, MF-Standard 0,5 bzw. 0,3) kontaminiert und danach 24 h bei 37 °C bebrütet. Bei Abstrichen von diesen Donatoren wurden Keimzahlen > 100.000 nachgewiesen.

In die Akzeptorschwänze wurden die Versuchssubstanzen zweimal täglich einmassiert.

Die Kontamination der Akzeptoren erfolgte durch Hautkontakt mit den Donatoren für 30 s bis 1 min auf der Schüttelmaschine bei 600 U/min. 2 h bzw. 24 h nach der Kontamination wurden die Donatoren auf Blut-Müller-Hinton-Agarplatten ausgestrichen. Nach 24 h Bebrütung der Agarplatten bei 37 °C wurden die Kolonien ausgezählt. Ergebnisse:

Beim Ausstreichen der Akzeptoren unmittelbar nach dem Hautkontakt sind die Platten vollständig bewachsen (Keimzahl > 10 000). Das gilt gleicherweise für vorbehandelte wie für Kontrollen ohne Vorbehandlung, d.h., die Akzeptoren kontaminiert. 2 h bzw.24 h sind dagegen nur noch geringen Keimzahlen bei den vorbehandelten Akzeptoren nachzuweisen, während die Keimzahlen bei den unbehandelten Kontrollen unverändert hoch sind.

### Legende zu den Figuren

- Figur 1:: Wirkung von Mikro- und Nanopartikeln aus der marinen Biomassen B30 (hergestellt nach Beispiel 1 und 2) im Vergleich zur synergistischen Kombination von B30 mit Vitamin C.
- Figur 2:: Nachweis der Unterbrechung von Infektketten, simuliert im Donator-Akzeptor-Modell, durch die erfindungsgemäße Zubereitung nach Beispiel 1 und 2

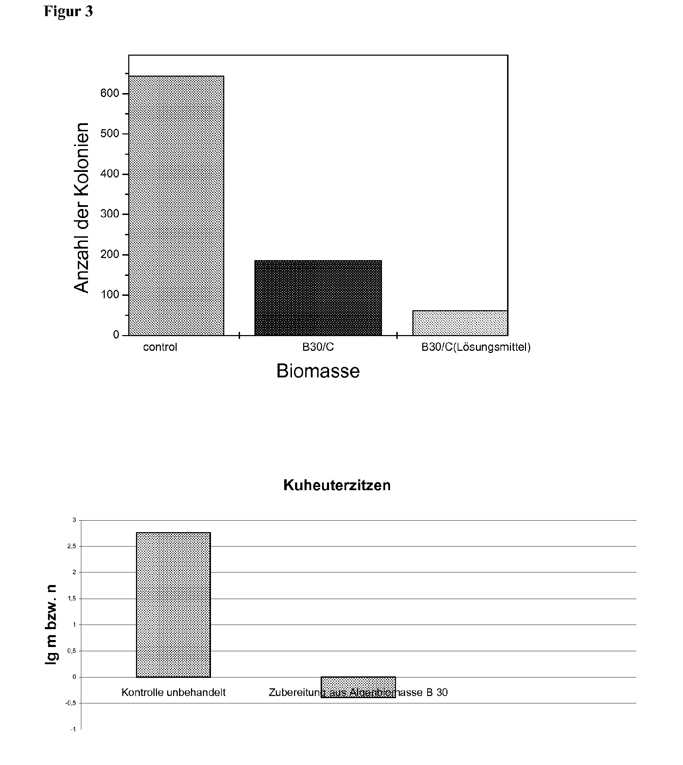

- Figur 3: Testung von Mikro- und Nanopartikeln "Biomasse B30/Vitamin C" hergestellt durch die erfindungsgemäße Zubereitung nach Beispiel 1, 2, 6 am Hängebauchschwein
- Figur 4:: Wirkung von Mikro- und Nanopartikeln hergestellt nach Beispiel 6

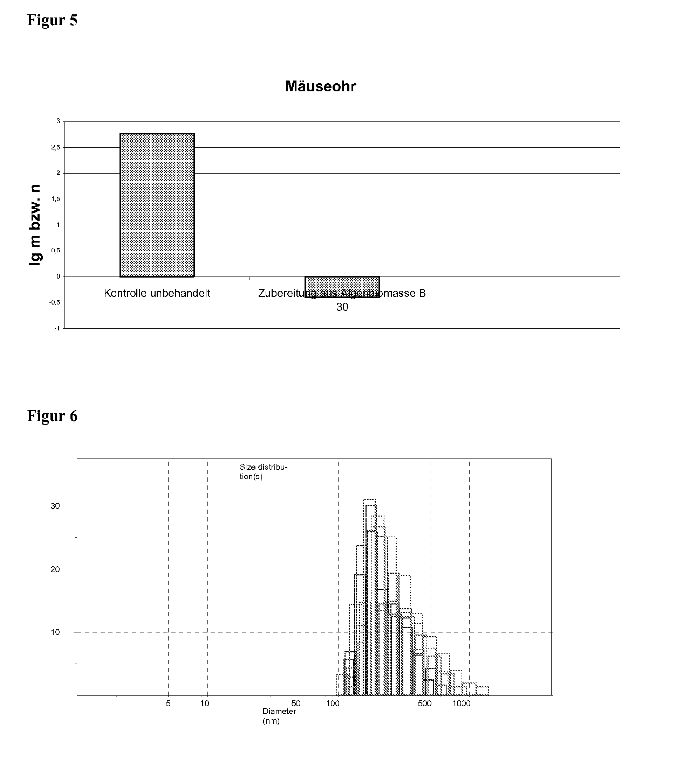

- Figur 5 :: Nachweis der Unterbrechung von Infektketten, simuliert im Donator-Akzeptor-Modell, durch die erfindungsgemäße Zubereitung nach Beispiel 6
- Figur 6:: Teilchengrößenverteilung Mikro- und Nanopartikeln, hergestellt nach Beispiel 3

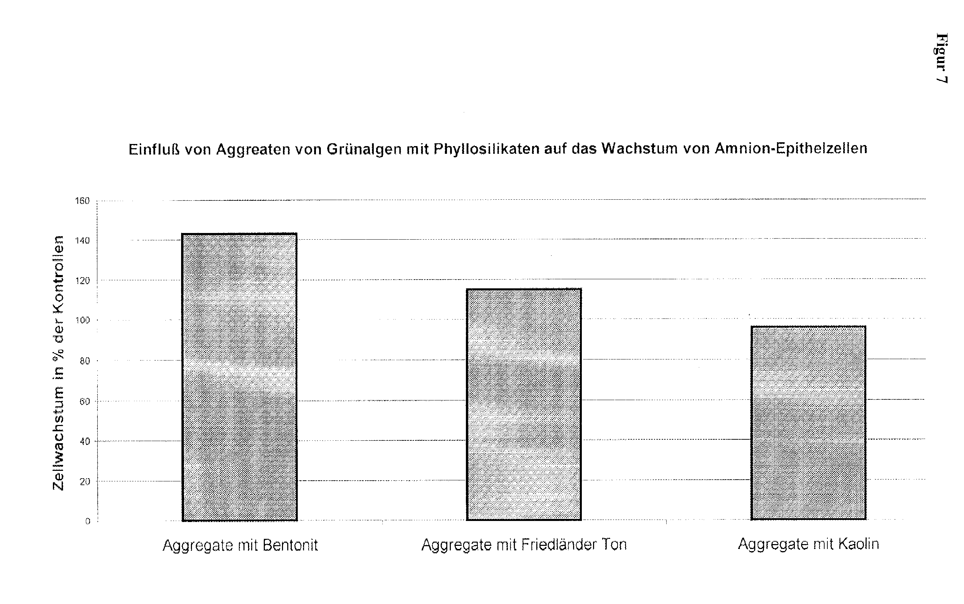

- Figur 7:: Einfluss von Aggregaten von Grünalgen mit Phyllosilikaten auf das Wachstum von Amnion-Epithelzellen.

## Patentansprüche

1. Verfahren zur Herstellung pharmazeutisch wirksamer Produkte aus Biomassen lipidhaltiger Mikro- und Markoalgen, mariner Pilze, Cyanobakterien oder mariner Bakterien, **dadurch gekennzeichnet, dass** die Biomassen durch Homogenisierung oder Emulsionsbildung in lipidhaltige Mikro- und Nanopartikel umgewandelt werden.

2. Verfahren nach Anspruch 1, **dadurch gekennzeichnet, dass** die lipidhaltigen Mikro- und Nanopartikel einen mittleren Durchmesser von 10 nm -10 µm besitzen.

3. Verfahren nach Anspruch 2, **gekennzeichnet durch** folgende Schritte:
- Erwärmen der Biomassen bis zur Verflüssigung der darin enthaltenen Fettsäuren
- ggf. Zusetzen eines oder mehrerer Wirkstoffe oder Zusätze
- Versetzen der Biomasse oder der beladenen Biomasse mit einem auf oberhalb der Schmelztemperatur der Fettsäuren erwärmten Tensid-Wasser-Gemisch und Vereinigung der beiden Phasen
- Herstellen einer Vorsuspension
- Hochdruckhomogenisierung in einem oder mehreren Homogenisationszyklen.

4. Verfahren nach Anspruch 3, **dadurch gekennzeichnet, dass** das Erwärmen der Biomassen und des Tensid-Wasser-Gemisches entfällt und die Wirkstoffe bei Raumtemperatur an die lipidhaltigen Mikroorganismen adsorbiert oder bei Zusatz einer geringen Wassermenge dispergiert werden.

5. Verfahren nach Anspruch 2, **gekennzeichnet durch** folgende Schritte:
- Suspendieren der Biomassen und ggf. der Zusätze in einem organischen Lösungsmittel und Vordispergieren dieses Gemisches
- Homogenisierung und Sprüh- oder Gefriertrocknung bzw. Rotationsverdampfung
- Redispergierung in einer wässrigen Tensidlösung
- erneute Dispergierung und Hochdruckhomogenisierung in einem oder mehreren Homogenisationszyklen.

6. Verfahren nach Anspruch 2, **gekennzeichnet durch** folgende Schritte:
- Emulsionsbildung aus Wasser und Biomasse sowie ggf. mit den Zusätzen
- Lösen der Emulsion in einem geeigneten organischen Lösungsmittel
- Hinzufügen eines wasserlöslichen Co-Tensids und Vordispergierung
- Hochdruckhomogenisierung und Entfernung des Lösungsmittels.

7. Verfahren nach Anspruch 1 oder 2, **dadurch gekennzeichnet, dass** als lipidhaltige Organismen
a) Cyanobakterien der Ordnung Oscillatoriales, insbesondere die Stämme SPH 03, SPH 04, SPH 05, SPH 06, SPH 09, SPH 10, SPH 11, SPH 12, SPH 13, SPH 14, SPH 20, SPH 21, SPH 22, SPH 23, SPH 25, SPH 26, SPH 29, SPH 32, SPH 34, SPH 37 und/oder
b) der Ordnung Nostocales, insbesondere die Stämme SPH 18,SPH 20, SPH 27, SPH 28, SPH 38 und/oder
c) der Ordnung Chroococcales, insbesondere die Stämme SPH 07a, SPH 07b, SPH 08, SPH 14, SPH 16, SPH 17, SPH 24, SPH 33, SPH 36, SPH 39, SPH 40, SPH 43 und/oder
d) der Ordnungen Stigonematales und/oder
e) Makroalgen der Gattungen Asparagopsis, Cystoseira, Codium, Dictyota, Dictyopteris, Enteromorpha, Fucus, Gelidium, Gracilaria, Gracilariopsis, Halopteris, Hypoglossum, Laurencia, Plocamium, Polyneura, Sargassum, Solieria, Ulva und/oder
f) Thraustochytriden der Gattungen Schizochytrium und Thraustochytrium und/oder
g) marine Bakterien der Gattungen Photobacterium, Shewanella und Colwellia eingesetzt werden.

8. Verfahren nach einem der Ansprüche 4 bis 7, **dadurch gekennzeichnet, dass** als Wirkstoffe oder Zusätze folgende Substanzen enthalten sind:
a) Vitamin C und/oder
b) Xanthon oder deren Derivate und/oder
c) Norlichhexanthon und/oder
d) Ubichinone der Kettenlänge n= 1 bis n = 15 und/oder
e) anorganische Thiocyanate und/oder
f) Hydrothiocyanate organischer Basen und/oder
g) Trihydroxybenzaldehyd oder dessen Derivate und/oder
h) DNA und/oder
i) Phyllosilikate, insbesondere Bentonit mit einem Durchmesser < 2 µm und/oder
j) dispersionsstabilisierende Substanzen.

9. Verwendung von Biomassen lipidhaltiger Mikro- und Markoalgen, mariner Pilze, Cyanobakterien oder mariner Bakterien, die durch Homogenisierung oder Emulsionsbildung in lipidhaltige Mikro- und Nanopartikel umgewandelt wurden, zur Herstellung von pharmazeutisch wirksamen Mitteln oder Nahrungsmitteln.

10. Verwendung nach Anspruch 9 zur Prophylaxe nosokomialer Infektionen.

11. Verwendung nach Anspruch 9 oder 10 zur Hemmung von multiresistenten Staphylokokken, insbesondere MRSA (methicillinresistente Staphylococcus aureus Stämme).

12. Verwendung nach Anspruch 9 zur Verbesserung der natürlichen Barrierefunktion der Haut und/oder zur Veränderungen des Hautmilieus.

13. Verwendung nach Anspruch 9
a) als Antibiotikaträger oder
b) zur dosierten Freisetzung antimikrobieller Wirkstoffe und gleichzeitiger Immunstimulation oder
c) in Slow-release-Systemen zur Prävention Implantat-assoziierter Infektionen oder
d) zur Stimulation von Leukozyten oder zur Aktivierung des retikoloendothelialen Systems oder
e) zur Imprägnierung von textilen und/oder auf Zellulosebasis hergestellten Materialien oder als Abdeckmaterialien zur Wundversorgung oder
f) zur Beschleunigung des Zellwachstums oder
g) zur gezielten Substituierung von Mangelzuständen.

14. Verwendung nach einem der Ansprüche 10 bis 13 in Kombination mit anderen pharmazeutisch wirksamen Mitteln.

15. Verwendung nach einem der Ansprüche 10 bis 14 in Form von Ölen, Sprays und/oder Salben.
